# EUROPEAN PATENT APPLICATION

(11) **EP 2 789 345 A1**
(43) Date of publication of application: **15.10.2014**
(21) Application number: 13425052.1
(22) Date of filing: 09.04.2013
(51) Int. Cl.: A61K 38/48, A61K 31/198, A61K 31/385

(54) **Composition for the treatment of endometriosis**

(71) Applicant: Pizeta Group S.r.l., 06135 Ponte San Giovanni (PG) (IT)
(72) Inventor: De Seta, Freancesco, 34151 Trieste (IT); Leonardi, Lucio, 06100 Perugia (IT)
(74) Representative: Pistolesi, Roberto

(57) **Abstract**

The present invention relates to a new composition, preferably with delayed release, containing N-acetyl cysteine, alpha-lipoic acid, and bromelain, in addition to the use thereof in the treatment of endometriosis, dysmenorrhoea, and/or pelvic pain.

## Description

The present invention relates to a new composition, preferably with delayed release, containing N-acetyl cysteine, alpha-lipoic acid, and bromelain, in addition to the use thereof in the treatment of endometriosis, dysmenorrhoea and/or pelvic pain.

### STATE OF THE ART

Endometriosis is a chronic, complex disease, which originates from the abnormal presence of tissues that usually line the inner wall of the uterus, otherwise known as the endometrium, in other organs, such as the ovaries, fallopian tubes, peritoneum, vagina, or intestines.

This results in internal bleeding, acute and chronic inflammation, and cicatrisation, which could lead to adhesions and infertility.

The disease usually manifests with acute and/or chronic pain, which can reach such levels as to have a disabling effect. These extremely characteristic symptoms tend to appear in particular during the menstrual cycle or during the intermenstrual period and worsen upon evacuation or urination. It is precisely this pro-inflammatory state that is responsible for the dysmenorrhoea, pain, and infertility of patients affected by the disease. The pathogenic cascade of inflammation is brought about by the enzyme COX (cyclooxygenase), which, from arachidonic acid, leads to the formation of prostanoids, the main actors of inflammation.

It has been highlighted how increased expression of the COX-2 enzyme in endometrial tissue causes the formation of PGE2 and PGF2 alpha prostaglandins as mediators of pain.

To date, no definitive endometriosis treatments have been identified yet. Until now, the management of this disease has concentrated on reducing symptoms or stopping the progression of the foci of endometriosis and has included medical and surgical treatments. The surgical treatment, carried out alone or in combination with medical treatment, is a common treatment for all stages of endometriosis. The aim of this treatment is to remove visible areas of endometriosis and restore the anatomy through lysis of adhesions. However, not everyone agrees that surgery alone gives greater relief from the symptoms of pain with respect to medical treatment. In fact, localisation, extent, and chronicity of the endometriotic process can lead to diverse clinical scenarios.

The medical treatments currently available for endometriosis aim to reduce the synthesis of oestrogen, induce atrophy of the endometrial implants, interrupt the stimulation/bleeding cycle, and inhibit the formation/progression of new endometriotic foci.

Oral contraceptives, androgenic agents (such as danazol), progestin agents (such as medroxyprogesterone acetate), and gonadotropin releasing hormone agonists (GnRH-a - such as leuprolide acetate, goserelin acetate, nafarelin acetate) have been used so far as the main form of treatment for endometriosis. Among these, GnRH agonists represent the most widespread medical treatment for endometriosis, since they are capable of establishing a state of "pharmacological menopause" and therefore an important state of hypoestrogenism. Danazol, progestational agents, GnRH-a, and oral contraceptives display, in the short term, comparable success rates as regards relieving the symptoms of endometriosis, but their effectiveness remains only partial in terms of reducing the size of endometriosis-related lesions (Endometriosis : ancient disease, ancient treatments. Nezhat C, Nezhat F, Nezhat C. Fertil Steril. 2012 Dec;98(6 Suppl):S1-62. doi: 10.1016/j.fertnstert.2012.08.001. Epub 2012 Oct 17. Systematic review of therapies for noncyclic chronic pelvic pain in women. Yunker A, Sathe NA, Reynolds WS, Likis FE, Andrews J. Obstet Gynecol Surv. 2012 Ju1;67(7):417-25. doi: 10.1097/OGX.0b013e31825cecb3. Review).

Not only do the short-term benefits of the conventional medical treatments involve side effects, they also do not offer a permanent solution to the risk of recurrence of the disease once the treatment is finished. The stigma of these side effects linked to conventional medicine has prompted several study groups to emphasize the role of non-conventional medicine. For example, although clinical trials on herbal formulas used in Chinese folk medicine (commonly prepared as both a boiled decoction and as dried extracts, or taken in pill form) have reported encouraging results as regards symptoms such as dysmenorrhoea, dysuria, dyschezia, and menorrhagia, such treatments have not been effective in permanently curing patients affected by the aforesaid disease.

Therefore, there is a need to identify an alternative treatment, which can reduce the pain symptoms, without side effects, and which is well tolerated by the patient.

### DESCRIPTION

Surprisingly, it has been found that a composition comprising N-acetyl cysteine, alpha-lipoic acid, and bromelain can be advantageously used in the treatment of endometriosis, dysmenorrhoea, and/or pelvic pain.

The term "treatment of endometriosis" according to the present invention means the reduction of the progression of the endometriotic mass and the reduction of the pain symptoms associated with the disease.

N-acetyl cysteine is a molecule which has the property of cleaving disulfide bonds, which explains why it is traditionally used as a mucolytic agent (it cleaves the disulfide bonds of mucoproteins) and as an antioxidant (for the regeneration of glutathione from GSSG to GSH). It has been highlighted that, since the state of inflammation in endometriosis is a chronic process, it also involves an increase in oxidative stress and up-regulation of the production of reactive oxygen species (ROS) which play not only a pro-inflammatory role but also the role of second messengers of cell proliferation and therefore a role in the evolution of endometriosis and the growth thereof in volumetric terms.

Since the use of N-acetyl cysteine in endometriosis can decrease the volume of the ectopic endometrium, it therefore allows the evolution of the disease to be stopped in much the same way as in conventional hormone treatment. However, unlike the aforesaid hormonal treatment, the strength of the N-acetyl cysteine is that it also impacts on the inflammation process in parallel, thereby simultaneously interrupting two mechanisms at their origins (More than antioxidant: N-acetyl-L-cysteine in a murine model of endometriosis. Pittaluga E, Costa G, Krasnowska E, Brunelli R, Lundeberg T, Porpora MG, Santucci D, Parasassi T. Fertil Steril. 2010 Dec;94(7):2905-8. doi: 10.1016/j.fertnstert.2010.06.038. Epub 2010 Jul 23. Reactive oxygen species controls endometriosis progression. Ngô C, Chéreau C, Nicco C, Weill B, Chapron C, Batteux F. Am J Pathol. 2009 Jul;175(1):225-34. Inhibition of proliferation of endometrial stromal cells by trichostatin A, RU486, CDB-2914, N-acetylcysteine, and ICI 182780. Wu Y, Guo SW. Gynecol Obstet Invest. 2006;62(4):193-205. Effects of oxidants and antioxidants on proliferation of endometrial stromal cells. Foyouzi N, Berkkanoglu M, Arici A, Kwintkiewicz J, Izquierdo D, Duleba AJ. Fertil Steril. 2004 Oct;82 Suppl 3:1019-22).

Alpha-lipoic acid (also known as thioctic acid) is a molecule composed of two disulfide groups which participate in redox reactions in the form of glutathione and ascorbic acid antioxidant agents. The importance of its antioxidant activity is bivalent: directly, it is an acceptor of radicals and indirectly, it activates other antioxidants, such as divalent ions (Fe²⁺, Mg²⁺, Cu²⁺).. Therefore, by reducing the levels of oxidative stress, this compound contributes, acting synergistically with the N-acetyl cysteine to reduce the proliferation of endometriotic cells (Alfa-lipoic acid controls tumor growth and modulates hepatic redox state in Ehrlich-ascites-carcinoma-bearing mice. Al Abdan M.Scientific WorldJournal. 2012;2012:509838. Epub 2012 May 1. Antioxidant therapy: current status and future prospects. Firuzi O, Miri R, Tavakkoli M, Saso L. Curr Med Chem. 2011;18(25):3871-88. Review).

As far as bromelain is concerned, this is a molecule which expresses proteolytic activity and is extracted from the fruit *Ananas comosus.* Already commonly known in folk medicine, this compound has anti-inflammatory and anti-edematous properties. Numerous studies have demonstrated a strong anti-inflammatory action of bromelain through selective inhibition of COX-2 and, in particular, *in vivo* data has demonstrated decreased production of PGE2 after taking bromelain (Treatment with oral bromelain decreases colonic inflammation in the IL-10-deficient murine model of inflammatory bowel disease. Hale LP, Greer PK, Trinh CT, Gottfried MR. Clin Immunol. 2005 Aug;116(2):135-42. In vivo and in vitro effects of bromelain on PGE(2) and SP concentrations in the inflammatory exudate in rats. Gaspani L, Limiroli E, Ferrario P, Bianchi M. Pharmacology. 2002 May;65(2):83-6).

Surprisingly, it has now been found that N-acetyl cysteine, alpha-lipoic acid, and bromelain, acting synergistically, impact on two specific aspects relating to the evolution of the pathology, namely the increase of the volume of the endometrium and the pain symptoms.

The present invention therefore relates to a composition containing N-acetyl cysteine, alpha-lipoic acid, and bromelain, and preferably, at least one physiologically acceptable excipient.

According to one embodiment of the present invention, the N-acetyl cysteine is present in the said composition in an amount by weight ranging from 35% to 70%, preferably from 40% to 60%, and more preferably from 44% to 52%, with respect to the total weight of the composition. Alpha-lipoic acid is present in the composition of the present invention in an amount by weight ranging from 10% to 25%, preferably from 12% to 20%, more preferably from 14 to 17%, with respect to the total weight of the composition.

Bromelain is present in the composition of the present invention in an amount by weight ranging from 0.1 % to 10%, preferably from 1% to 5%, more preferably from 1.5% to 2%, with respect to the total weight of the composition.

According to the present invention, the aforesaid composition may be formulated in oral form, preferably in the form of a tablet or capsule, more preferably in the form of a coated tablet or capsule; the preferred coating according to the present invention is a gastro-resistant coating.

According to one aspect of the present invention, the composition has a delayed release and includes a core containing N-acetyl cysteine, alpha-lipoic acid, bromelain, at least one physiologically acceptable excipient, and a gastro-resistant coating.

According to a particularly preferred embodiment, the said delayed-release composition comprises:
a) a core consisting of: a controlled-release matrix in which alpha-lipoic acid is dispersed; N-acetyl cysteine; bromelain; at least one physiologically acceptable excipient;
b) a gastro-resistant coating.

According to the present invention, the term "physiologically acceptable excipient" means a substance devoid of any pharmacological effect of its own and which does not produce adverse reactions when administered to a mammal, preferably a human.

According to the present invention, the term "delayed-release formulation or composition" means a form of administration that allows the active ingredient contained therein to be released after a predetermined time. According to the present invention, the term "matrix" means a matrix of a polymeric nature in which the active ingredient is dispersed in a homogenous manner.

According to the present invention, the term "controlled-release matrix" means a matrix that releases the active ingredient dispersed therein slowly over time and allows blood or tissue levels to be kept constant over a given period of time.

According to the present invention, the term "gastro-resistant coating" means a coating that allows the composition to cross the gastric tract without being damaged and then release the active ingredient or ingredients within the intestine.

In particular, the controlled-release matrix in which the alpha-lipoic acid is dispersed is composed of at least one polymer chosen from ethyl cellulose, methyl cellulose, and hydropropylmethyl cellulose. Said polymer is preferably ethyl cellulose.

The excipients usable are preferably chosen from microcrystalline cellulose, cross-linked carboxymethyl cellulose, lactose, corn starch, silicon dioxide, magnesium stearate, tribasic calcium phosphate, talc and/or polyvinylpyrrolidone; said excipients are preferably microcrystalline cellulose, silicon dioxide, talc, and magnesium stearate.

Preferably, excipients usable for the purposes of the present invention, including the polymers of the matrix, are present in an amount by weight ranging from 15% to 50%, more preferably from 20% to 40%, still more preferably from 25 to 35%, with respect to the total weight of the composition.

Suitable gastro-resistant coatings according to the present invention may be chosen from polymers and copolymers of acrylic and methacrylic acid, polyvinyl acetate phthalate, modified starch, glycerol, talc, or mixtures thereof. The gastro-resistant coating may also include one or more dyes. The gastro-resistant coating according to the present invention is present in said composition in an amount by weight ranging from 5% to 20%, preferably from 7% to 15%, with respect to the total weight of the composition.

The gastro-resistant coating prevents the gastric absorption of the active ingredients, which involves a substantial first-pass hepatic metabolism effect and thus a poor bioavailability of said ingredients. Moreover, this coating preserves the active ingredients against rapid oxidation, in addition to masking the bad odour released from the N-acetyl cysteine.

The matrix in which, according to one aspect of the invention, the alpha-lipoic acid is dispersed allows the controlled release thereof (about 20-25% every two hours over a total of eight hours). Said controlled release allows to administer the composition of the present invention with a lower number of daily administrations, despite the very short half-life (15-20 minutes) of alpha-lipoic acid.

Such a mode of administration therefore involves fewer side effects and the composition of the present invention results more bioavailable with respect to conventional treatments.

A further aim of the present invention is the use of the aforesaid composition in the treatment of endometriosis, dysmenorrhoea, and/or pelvic pain, the recipient of the treatment preferably being a mammal, preferably a human, and even more preferably female.

According to one embodiment of the present invention, said composition is administered from once to four times a day, preferably twice a day.

The compositions of the present invention can be prepared with conventional methods well known to persons skilled in the art.

The following examples are given for illustrative and non-limiting purpose of the present invention.

### EXPERIMENTAL SECTION

### Example 1 (table)

| | |
|---|---|
| Matris® Thioctic (equivalent to 200 mg of alpha-lipoic acid)* | 285 mg |
| N-acetyl cysteine | 600 mg |
| Bromelain 2500 GDU | 25 mg |
| Microcrystalline cellulose | 235 mg |
| Silicon dioxide | 10 mg |
| Vegetable magnesium stearate | 10 mg |
| Talc | 10 mg |

The tablet is prepared by mixing and subsequent compressing the components.
* This is a matrix containing alpha-lipoic acid, ethyl cellulose, talc, and silicon dioxide, marketed by IPS International Products & Services S.r.l.

### CLINICAL DATA

Five women with stage I-II endometriosis who had already undergone surgical treatment, wanted children and stopped chronic treatment with NSAIDs (non-steroidal anti-inflammatory drugs), but suffering from persistent, chronic pelvic pain were treated with the tablet from Example 1, three times a day for three 30-day cycles without interruption, following conventional treatment (NSAIDs) in the month preceding the start of administration of the product.

Five women with a history of primary dysmenorrhoea, who wanted children and had refused to take NSAIDs were treated with the composition from Example 1, three times a day for five days per month during the perimenstrual period.

### FOLLOW-UP and OBSERVATIONAL RESULTS

All the women reported a significant reduction in pain symptoms (meaning thereby the level of abdominal-pelvic pain), as well as being able to carry out and resume normal work activity, and an enhancement of regular sexual activity which had previously affected by deep dyspareunia. Non-observational data based on more larger, homogeneous collection of randomised case studies, selected based on the genesis of chronic pelvic pain (primary dysmenorrhoea, endometriosis, premenstrual syndrome, etc.) will be able to confirm these observational findings and above all validate the possible effectiveness of the product.

## Claims

1. A composition containing N-acetyl cysteine, alpha-lipoic acid, and bromelain.

2. A composition according to claim 1, **characterised in that** N-acetyl cysteine is in an amount by weight ranging from 35% to 70%, preferably from 40% to 60%, with respect to the total weight of the composition.

3. A composition according to claim 2, **characterised in that** N-acetyl cysteine is in an amount by weight ranging from 44% to 52%, with respect to the total weight of the composition.

4. A composition according to claim 1, **characterised in that** alpha-lipoic acid is in an amount by weight ranging from 10% to 25%, preferably from 12% to 20%, with respect to the total weight of the composition.

5. A composition according to claim 4, **characterised in that** alpha-lipoic acid is in an amount by weight ranging from 14% to 17%, with respect to the total weight of the composition.

6. A composition according to claim 1, **characterised in that** bromelain is in an amount by weight ranging from 0.1% to 10%, preferably from 1% to 5%, with respect to the total weight of the composition.

7. A composition according to claim 6, **characterised in that** bromelain is in an amount by weight ranging from 1.5% to 2%, with respect to the total weight of the composition.

8. A composition according to any one of the previous claims, **characterised in that** said composition is formulated in an oral form, preferably in the form of a tablet or capsule.

9. A composition according to any one of the previous claims, **characterised in that** said composition is gastro-resistant coated.

10. A composition according to claim 9, **characterised in that** said gastro-resistant coating is present in an amount ranging from 5% to 20%, preferably from 7% to 15%, with respect to the total weight of the composition.

11. A composition, according any of the previous claims, **characterised in that** said composition comprises at least one physiologically acceptable excipient.

12. A composition according to claim 11, **characterised in that** said at least one excipient is present in an amount ranging from 15% to 50%, more preferably from 20% to 40%, and still more preferably from 25 to 35%, with respect to the total weight of the composition

13. A composition according to any one of the previous claims, for use in the treatment of endometriosis, dysmenorrhoea and/or pelvic pain.

14. A composition for use according to claim 13, **characterised in that** the recipient of the treatment is a mammal, preferably a human, and still more preferably female.

15. A composition for use according to claim 13, **characterised in that** said composition is administered once to four times a day, preferably twice a day.
